(19) Europäisches Patentamt
European Patent Office
Office européen des brevets

(11) EP 4 763 218 A1

(12) EUROPEAN PATENT APPLICATION

(43) Date of publication:
**24.06.2026 Bulletin 2026/26**

(21) Application number: **25222475.3**

(22) Date of filing: **11.12.2025**

(51) International Patent Classification (IPC):
***A61K 38/18*** *(2006.01)* ***A61K 35/19*** *(2015.01)*
***A61Q 19/02*** *(2006.01)* ***A61P 17/00*** *(2006.01)*

(52) Cooperative Patent Classification (CPC):
**A61P 17/00; A61K 8/983; A61K 35/19; A61K 38/1841; A61K 38/1858; A61Q 19/02;**
A61K 2035/124

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **19.12.2024 US 202463736121 P**

(71) Applicants:
- **Spirit Scientific Co. LTD.**
  **New Taipei City 221416 (TW)**
- **Lin, Dao Lung, Steven**
  **New Taipei City 221416 (TW)**

(72) Inventors:
- **Lin, Dao Lung Steven**
  **221416 New Taipei City (TW)**
- **CHEN, Chin Ho**
  **221416 New Taipei City (TW)**

(74) Representative: **Ter Meer Steinmeister & Partner Patentanwälte mbB**
**Nymphenburger Straße 4**
**80335 München (DE)**

# (54) COMPOSITION COMPRISING PLATELETS AND/OR GROWTH FACTORS FOR USE IN THE INHIBITION OF MELANIN GENERATION

(57) A composition for inhibiting generation of melanin and the use thereof is provided, the composition includes platelets and/or growth factors isolated from blood, the composition for inhibiting generation of melanin has the abilities for inhibiting activity of tyrosinase and alleviating inflammation, and the composition for inhibiting generation of melanin is able to be used to manufacture of preparation for improving skin pigmentation or alleviating melasma.

EP 4 763 218 A1

## Description

### FIELD OF THE INVENTION

The present invention relates to a composition for inhibiting generation of melanin and the use thereof, in particular, to a composition comprising platelets and/or growth factors isolated from blood for inhibiting generation of melanin and the use for manufacturing of a preparation for inhibiting generation of melanin in a subject.

### BACKGROUND OF THE INVENTION

Skin is the largest organ in the human body and is divided into epidermis, which is composed of stratum corneum and basal layer; dermis and subcutaneous tissue. The color of skin is affected by a variety of pigments, including melanin, carotene, and hemoglobin. Melanin is produced by melanocytes, which are mainly distributed in the stratum basale.

Tyrosinase is an oxidase and a rate-limiting enzyme that regulates generation of melanin. When melanocytes are stimulated, they activate the activity of tyrosinase, which initiates a series of chemical reactions and finally produces melanin. When melanosomes containing a large amount of melanin are released into keratinocytes, they cause the skin to darken or form various spots (e.g., nevus, melasma). Therefore, inhibiting the production and activity of tyrosinase is considered a key step in reducing melanin generation, but when melanin is not produced, it causes vitiligo and leads to additional skin conditions.

Currently, commonly used whitening ingredients on the market are mainly added to cosmetics or skin care products to inhibit melanin formation and prevent pigment spots. If it is not added according to the concentration limit, it may cause allergies, apoptosis or death of melanocytes. Therefore, there is still a need for a composition or formulation that can reduce melanin generation without causing melanocyte apoptosis or death.

### DETAIL DESCRIPTION OF THE INVENTION

The purpose of the present invention is to provide a composition for inhibiting generation of melanin, which comprises platelets and/or growth factors isolated from blood.

In one embodiment, the composition comprises platelets or growth factors isolated from blood, preferably, the composition comprises platelets isolated from plasma.

The composition preferably comprises $2.5\times10^7$ ~ $10\times10^9$ platelets.

The composition preferably comprises $2.5\times10^7$ ~ $10\times10^8$ platelets.

The composition preferably comprises $5\times10^7$ ~ $10\times10^8$ platelets.

In another embodiment, the composition comprises platelet-derived growth factor-BB (PDGF-BB) and/or transforming growth factor (TGF-β).

In a preferred manner, the composition comprises PDGF-BB with a concentration of 0.1 ng/mL ~ 5 ng/mL.

In a preferred manner, the composition comprises PDGF-BB with a concentration of 0.1 ng/mL ~ 3 ng/mL.

In a preferred manner, the composition comprises PDGF-BB with a concentration of 0.25 ng/mL ~ 2 ng/mL.

In a preferred manner, the composition comprises PDGF-BB with a concentration of 0.25 ng/mL ~ 1 ng/mL.

In a preferred manner, the composition comprises TGF-β with a concentration of 0.5 ng/mL ~ 50 ng/mL.

In a preferred manner, the composition comprises TGF-β with a concentration of 0.5 ng/mL ~ 30 ng/mL.

In a preferred manner, the composition comprises TGF-β with a concentration of 1 ng/mL ~ 20 ng/mL.

In a preferred manner, the composition comprises TGF-β with a concentration of 1 ng/mL ~ 10 ng/mL.

In another embodiment, the composition for inhibiting generation of melanin used in the present invention is freeze-dried to form a dry powder.

In a preferred manner, the dry powder of the composition comprises $4\times10^8$ ~ $1.5\times10^{11}$ platelets per gram.

In a preferred manner, the dry powder of the composition comprises $4\times10^8$ ~ $1.5\times10^{10}$ platelets per gram.

In a preferred manner, the dry powder of the composition comprises $8\times10^8$ ~ $1.5\times10^{10}$ platelets per gram.

In a preferred manner, the dry powder of the composition comprises PDGF-BB with a concentration of 15 ~ 850 ng per gram.

In a preferred manner, the dry powder of the composition comprises PDGF-BB with a concentration of 15 ~ 500 ng per gram.

In a preferred manner, the dry powder of the composition comprises PDGF-BB with a concentration of 35 ~ 350 ng per gram.

In a preferred manner, the dry powder of the composition comprises PDGF-BB with a concentration of 35 ~ 200 ng per gram.

In a preferred manner, the dry powder of the composition comprises TGF-β with a concentration of 75 ~ 8,500 ng per gram.

In a preferred manner, the dry powder of the composition comprises TGF-β with a concentration of 75 ~ 5,000 ng per gram.

In a preferred manner, the dry powder of the composition comprises TGF-β with a concentration of 150 ~ 3,500 ng per gram.

In a preferred manner, the dry powder of the composition comprises TGF-β with a concentration of 150 ~ 2,000 ng per gram.

The another purpose of the present invention is to provide a composition for use in alleviating skin pigmentation in a subject

In an embodiment, the pigmentation is caused by inflammation of cells.

In an embodiment, the pigmentation is induced by tyrosinase.

The another purpose of the present invention is to provide a composition for use in alleviating melasma in a subject

The composition for inhibiting generation of melanin in the present invention comprises $1\times10^7$, $1.25\times10^7$, $1.5\times10^7$, $1.75\times10^7$, $2\times10^7$, $2.25\times10^7$, $2.5\times10^7$, $2.75\times10^7$, $3\times10^7$, $3.25\times10^7$, $3.5\times10^7$, $3.75\times10^7$, $4\times10^7$, $4.25\times10^7$, $4.5\times10^7$, $4.75\times10^7$, $5\times10^7$, $5.25\times10^7$, $5.5\times10^7$, $5.75\times10^7$, $6\times10^7$, $6.25\times10^7$, $6.5\times10^7$, $6.75\times10^7$, $7\times10^7$, $7.25\times10^7$, $7.5\times10^7$, $7.75\times10^7$, $8\times10^7$, $8.25\times10^7$, $8.5\times10^7$, $8.75\times10^7$, $9\times10^7$, $9.25\times10^7$, $9.5\times10^7$, $9.75\times10^7$, $1\times10^8$, $1.25\times10^8$, $1.5\times10^8$, $2\times10^8$, $3\times10^8$, $4\times10^8$, $5\times10^8$, $6\times10^8$ $7\times10^8$, $8\times10^8$, $9x10^8$, $1\times10^9$, $2\times10^9$, $3\times10^9$, $4\times10^9$, $5\times10^9$, $6\times10^9$, $7\times10^9$, $8\times10^9$, $9\times10^9$, $1\times10^{10}$ platelets.

In the present invention, the composition for inhibiting generation of melanin comprises a growth factor isolated from blood, the growth factor is obtained by centrifuging whole blood samples, such as whole blood from non-human mammals or human, to obtain a high-concentration platelet-rich plasma (PRP), activators such as calcium chloride ($CaCl_2$), thrombin, collagen, adenosine diphosphate (ADP), etc. are added as needed, followed by further centrifugation.

In the present invention, the concentration of PDGF-BB is preferably 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5 ng/mL.

In the present invention, the concentration of TGF-β is preferably 0.1, 0.2, 0.3, 0.4, 0.5, 0.6, 0.7, 0.8, 0.9, 1, 1.1, 1.2, 1.3, 1.4, 1.5, 1.6, 1.7, 1.8, 1.9, 2, 2.1, 2.2, 2.3, 2.4, 2.5, 2.6, 2.7, 2.8, 2.9, 3, 3.1, 3.2, 3.3, 3.4, 3.5, 3.6, 3.7, 3.8, 3.9, 4, 4.1, 4.2, 4.3, 4.4, 4.5, 4.6, 4.7, 4.8, 4.9, 5, 5.1, 5.2, 5.3, 5.4, 5.5, 5.6, 5.7, 5.8, 5.9, 6, 6.1, 6.2, 6.3, 6.4, 6.5, 6.6, 6.7, 6.8, 6.9, 7, 7.1, 7.2, 7.3, 7.4, 7.5, 7.6, 7.7, 7.8, 7.9, 8, 8.1, 8.2, 8.3, 8.4, 8.5, 8.6, 8.7, 8.8, 8.9, 9, 9.1, 9.2, 9.3, 9.4, 9.5, 9.6, 9.7, 9.8, 9.9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 50 ng/mL.

The composition of the present invention can be used as injectable or topical preparation, wherein a suitable skin matrix or injectable carrier, or an aqueous solution, non-aqueous solvent, suspension, emulsion or freeze-dried formulation, etc. may be used in the preparation, and be sterilized according to known methods.

The composition of the present invention may be combined with its pharmaceutically acceptable solvent, carrier or excipient to form a preparation. Examples of solvents include, but are not limited to, water, saline, sterile water, deionized water, glycerol, and so on.

The composition of the present invention may further contain additional raw materials, such as antimicrobials or preservatives.

## BRIEF DESCRIPTION OF DRAWINGS

Figure 1 shows result of the effect of different concentration of platelet-derived growth factor-BB (PDGF-BB) in the composition of the present invention on melanocyte proliferation.

Figure 2 shows result of the effect of different concentration of platelet-derived growth factor-BB (PDGF-BB) in the composition of the present invention on melanin generation.

Figure 3 shows result of different concentration of platelet-derived growth factor-BB (PDGF-BB) in the composition of the present invention inhibiting the generation of melanin.

Figure 4 shows result of different concentration of transforming growth factor (TGF-β) in the composition of the present invention inhibiting the generation of melanin.

Figure 5 shows result of different amounts of platelets in the composition of the present invention inhibiting the generation of melanin.

Figure 6 shows result of different amounts of platelets in the composition of the present invention and different whitening ingredients inhibiting inflammation of melanocytes.

Figure 7 shows result of different amounts of platelets in the composition of the present invention and different whitening ingredients inhibiting the activity of tyrosinase.

## EMBODIMENTS

It should be understood that the detailed description of the embodiment is intended to illustrate the better embodiment of the present invention and not to limit the scope of the present invention.

### 1. Preparation of enriched platelet-derived growth factor-BB (PDGF-BB) growth factor dry powder

The growth factor of the present invention is isolated from the whole blood of human or non-human mammals.
250 milliliters (mL) of whole blood was put into a blood bag containing an anticoagulant and centrifuged at 300 ~ 1,500×g for 3 to 10 minutes to obtain platelet-rich plasma (PRP), also known as plasma layer solution.
Calcium chloride ($CaCl_2$) activator was added to the PRP, centrifugation was performed again to remove the supernatant, so that the proteins of plasma were completely removed.
Same volume of water for injection as the removed supernatant was added, the growth factors released from activated platelet were obtained. These were then aliquoted into sample vials at 1 mL per vial. The vials were then freeze-dried to obtain the growth factor powder.

### 2-1. Preparation of melanocyte-specific culture medium containing enriched platelet-derived growth factor-BB (PDGF-BB) dry powder

1 mL of melanocyte-specific basal cell culture medium (including melanocyte-specific growth factor kit) was used to dissolve growth factor dry powder to prepare dry powder medium. Each gram of growth factor dry powder contains 15 ~ 850 ng of PDGF-BB. The melanocyte-specific basal cell culture medium and dry powder medium were mixed in a certain proportion, then analyzed and evaluated by enzyme-linked immunosorbent assay (ELISA) to obtain enriched PDGF-BB dry powder culture medium with the final PDGF-BB concentration of 0 nanograms per milliliter (0 ng/mL), 0.12, 0.25, 0.5, 1.0, 2.0 ng/mL.

### 2-2. The effect of platelet-derived growth factor-BB (PDGF-BB) on melanocyte

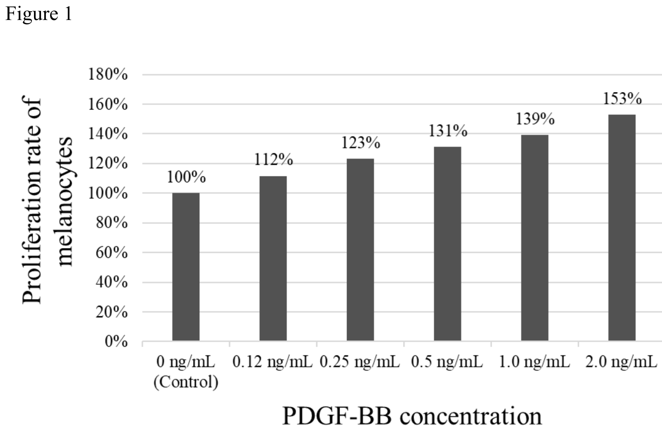

The 96-well plate was divided into untreated wells (control group wells, 0 ng/mL PDGF-BB), and growth factor wells with 0.12, 0.25, 0.5, 1.0, and 2.0 ng/mL PDGF-BB, with 3 replicates per well.
Human Epidermal Melanocytes (HEM; Purchased from ATCC, product number PCS-200-012) were cultured in melanocyte-specific basal cell culture medium (purchased from ATCC, product number PCS-200-030) containing melanocyte-special growth factor kit (purchased from ATCC, product number PCS-200-041) and then replaced with fresh culture medium for later use.
HEM cells were added to untreated wells (control wells), 0.12, 0.25, 0.5, 1.0, and 2.0 ng/mL PDGF-BB growth factor wells, each well contained the same amounts of HEM cells, cells were cultured in an incubator at 37°C and 5% carbon dioxide ($CO_2$) for 24 hours. Remove the old culture medium (used medium), enriched PDGF-BB dry powder culture medium corresponding to each group was added into the corresponding well and continued to incubate for 48 hours.
100 microliters (μL) of CCK-8 reagent (DOJINDO, CK-04-01) was added into the cells of the growth factor wells of 0 (control wells), 0.12, 0.25, 0.5, 1.0, and 2.0 ng/mL PDGF-BB, separately, placed in an incubator at 37°C and 5% $CO_2$ for 1-3 hours. After the reaction was completed, the cell culture suspension from each well was aspirated, transferred into a microcentrifuge tube, and analyzed by using a multi-function microplate spectrophotometer (Varioskan LUX™, Thermo Scientific™) with absorbance value of 450 nm. The regression curve of absorbance value and cell number was established according to the CCK-8 reagent product manual, and the absorbance value was then converted to the number of viable cells.
The experimental results are shown in table 1 and Figure 1. The number of melanocytes in the control group is set as a benchmark. The results show that PDGF-BB dose-dependently promotes normal growth of melanocytes.

Table 1

| PDGF-BB concentration (ng/mL) | 0 (control) | 0.12 | 0.25 | 0.5 | 1.0 | 2.0 |
|---|---|---|---|---|---|---|

(continued)

| Proliferation rate of melanocytes (%) | 100% | 112% | 123% | 131% | 139% | 153% |
|---|---|---|---|---|---|---|

**2-3. The effect of platelet-derived growth factor-BB (PDGF-BB) on generation of melanin of melanocyte**

The 96-well plate was divided into untreated wells (control group wells, 0 ng/mL PDGF-BB), and growth factor wells with 0.12, 0.25, 0.5, 1.0, and 2.0 ng/mL PDGF-BB, with 3 replicates per well.

Human Epidermal Melanocytes (HEM; Purchased from ATCC, product number PCS-200-012) were cultured in melanocyte-specific basal cell culture medium (purchased from ATCC, product number PCS-200-030) containing melanocyte-special growth factor kit (purchased from ATCC, product number PCS-200-041) and then replaced with fresh culture medium for later use.

HEM cells were added to untreated wells (control wells, 0 ng/mL PDGF-BB), 0.12, 0.25, 0.5, 1.0, and 2.0 ng/mL PDGF-BB growth factor wells, each well contained the same amounts of HEM cells, cells were cultured in an incubator at 37°C and 5% carbon dioxide ($CO_2$) for 24 hours. Remove the old culture medium (used medium), enriched PDGF-BB dry powder culture medium corresponding to each group was added into the corresponding well and continued to incubate for 48 hours.

500 μL of TrypLE reagent (Gibco, A1285901) was added into the cells of the growth factor wells of 0 (control wells), 0.12, 0.25, 0.5, 1.0, and 2.0 ng/mL PDGF-BB, separately, placed in an incubator at 37°C and 5% $CO_2$ for 3-5 minutes. After the reaction was completed, 500 μL of melanocyte-specific basal cell culture medium was added into each well to terminate the reaction of TrypLE reagent.

Cell fluid in each well was transferred into a microcentrifuge tube and centrifuged at 1,000-3,000 rpm (preferably, 2,000 rpm) for 10-30 minutes (preferably, 20 mins) with a microcentrifuge (Kubota, 3520) to remove the supernatant, and cell pellet was left. 110 μL of NaOH (Sigma, S5881) at a concentration of 1N was added into each tube for 10-20 minutes. After the reaction was completed, each mixture was transferred into a microcentrifuge tube and then analyzed using a multi-function microplate spectrophotometer (Varioskan LUX™, Thermo Scientific™) with absorbance value of 475 nm to obtain the absorbance of melanin.

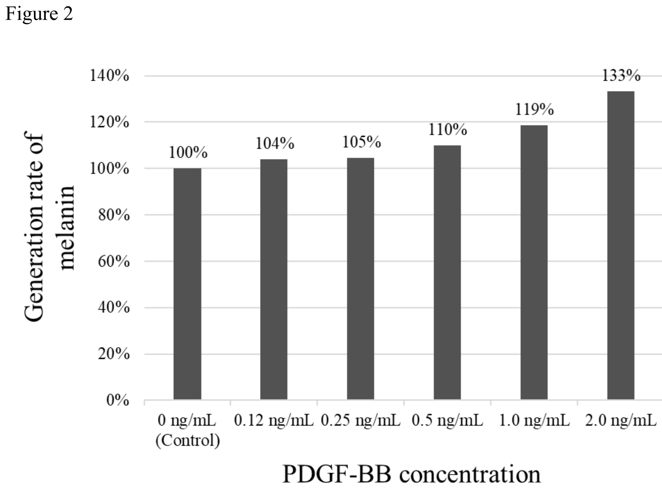

The experimental results are shown in table 2 and Figure 2. The amount of melanin in the control group is set as a benchmark. The results show that melanin is increased simultaneously with the growth of melanocytes under the action of PDGF-BB.

Table 2

| PDGF-BB concentration (ng/mL) | 0 (control) | 0.12 | 0.25 | 0.5 | 1.0 | 2.0 |
|---|---|---|---|---|---|---|
| Generation rate of melanin (%) | 100% | 104% | 105% | 110% | 119% | 133% |

**2-4. The inhibition effect of platelet-derived growth factor-BB (PDGF-BB) on generation of melanin**

Based on the results of the above experiments, the inhibition rate of melanin generation per unit of melanocytes was calculated as follows:

Inhibition rate of melanin generation (%) = (1 - the amount of melanin per unit of cell (tested group)) ÷ the amount of melanin per unit of cell (control group) × 100%

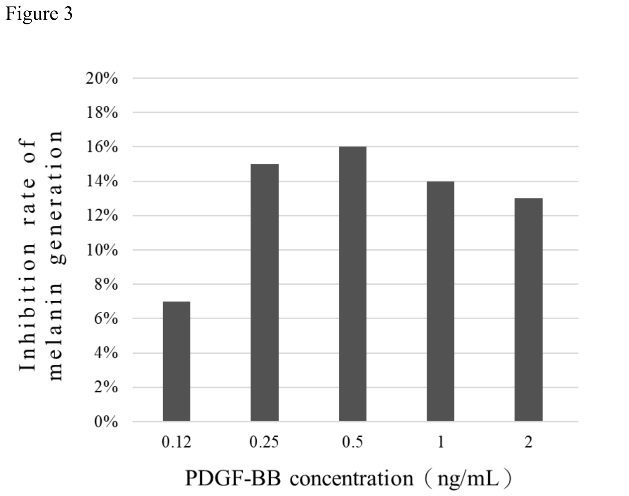

The experimental results are shown in table 3 and Figure 3. The amount of melanin in the control group is set as a benchmark. After calculations, it was found that although melanin increased simultaneously with the growth of melanocytes under the action of PDGF-BB, PDGF-BB actually inhibited the growth of melanin in single melanocytes. When the PDGF-BB concentration reaches 0.12 ng/mL, the inhibition rate of melanin generation reaches 7%; when the PDGF-BB concentration reaches 0.25 ng/mL or more, the inhibition rate of melanin generation reaches 10% or more, and when the PDGF-BB concentration is in the range of 0.25 ng/mL to 1 ng/mL, the inhibition rate of melanin generation reaches 14% or even higher.

Table 3

| PDGF-BB concentration (ng/mL) | Melanin/cell | Inhibition rate of melanin generation (%) |
|---|---|---|
| 0 (control) | 1 | 0% |
| 0.12 | 0.93 | 7% |

(continued)

| PDGF-BB concentration (ng/mL) | Melanin/cell | Inhibition rate of melanin generation (%) |
|---|---|---|
| 0.25 | 0.85 | 15% |
| 0.5 | 0.84 | 16% |
| 1.0 | 0.86 | 14% |
| 2.0 | 0.87 | 13% |

**3. Preparation of enriched transforming growth factor beta (TGF-β) growth factor dry powder**

The growth factor of the present invention is isolated from the whole blood of human or non-human mammals.

250 milliliters (mL) of whole blood was put into a blood bag containing an anticoagulant and centrifuged at 300 ~ 1,500×g for 3 to 10 minutes to obtain platelet-rich plasma (PRP), also known as plasma layer solution.

Thrombin activator was added to the PRP, centrifugation was performed again to remove the supernatant, so that the proteins of plasma were completely removed.

Same volume of water for injection as the removed supernatant was added, the growth factors released from activated platelet were obtained. These were then aliquoted into sample vials at 1 mL per vial. The vials were then freeze-dried to obtain the growth factor powder.

**4-1. Preparation of melanocyte-specific culture medium containing enriched transforming growth factor beta (TGF-β) dry powder**

1 mL of melanocyte-specific basal cell culture medium (including melanocyte-specific growth factor kit) was used to dissolve growth factor dry powder to prepare dry powder medium. Each gram of growth factor dry powder contains 75 ~ 8,500 ng of TGF-β. The melanocyte-specific basal cell culture medium and dry powder medium were mixed in a certain proportion, then analyzed and evaluated by enzyme-linked immunosorbent assay (ELISA) to obtain enriched TGF-β dry powder culture medium with the final TGF-β concentration of 0, 0.5, 1.0, 5.0, 10.0, 20.0 ng/mL.

**4-2. The inhibition effect of TGF-β on generation of melanin**

The 96-well plate was divided into untreated wells (control group wells, 0 ng/mL TGF-β), and growth factor wells with 0.5, 1.0, 5.0, 10.0, and 20.0 ng/mL TGF-β, with 3 replicates per well.

Human Epidermal Melanocytes (HEM; Purchased from ATCC, product number PCS-200-012) were cultured in melanocyte-specific basal cell culture medium (purchased from ATCC, product number PCS-200-030) containing melanocyte-special growth factor kit (purchased from ATCC, product number PCS-200-041) and then replaced with fresh culture medium for later use.

HEM cells were added to untreated wells (control wells, 0 ng/mL TGF-β), 0.5, 1.0, 5.0, 10.0, and 20.0 ng/mL TGF-β growth factor wells, each well contained the same amounts of HEM cells, cells were cultured in an incubator at 37°C and 5% carbon dioxide ($CO_2$) for 24 hours. Remove the old culture medium (used medium), enriched TGF-β dry powder culture medium corresponding to each group was added into the corresponding well and continued to incubate for 48 hours.

500 μL of TrypLE reagent (Gibco, A1285901) was added into the cells of the growth factor wells of 0 (control wells), 0.5, 1.0, 5.0, 10.0, and 20.0 ng/mL TGF-β, separately, placed in an incubator at 37°C and 5% $CO_2$ for 3-5 minutes. After the reaction was completed, 500 μL of melanocyte-specific basal cell culture medium was added into each well to terminate the reaction of TrypLE reagent.

Cell fluid in each well was transferred into a microcentrifuge tube and centrifuged at 1,000-3,000 rpm (preferably, 2,000 rpm) for 10-30 minutes (preferably, 20 mins) with a microcentrifuge (Kubota, 3520) to remove the supernatant, and cell pellet was left. 110 μL of NaOH (Sigma, S5881) at a concentration of 1N was added into each tube for 10-20 minutes. After the reaction was completed, each mixture was transferred into a microcentrifuge tube and then analyzed using a multi-function microplate spectrophotometer (Varioskan LUX™, Thermo Scientific™) with absorbance value of 475 nm to obtain the absorbance of melanin.

The inhibition rate of melanin generation per unit of melanocytes was calculated as follows:

Inhibition rate of melanin generation (%) = (1 - the amount of melanin per unit of cell (tested group)) ÷ the amount of melanin per unit of cell (control group) × 100%

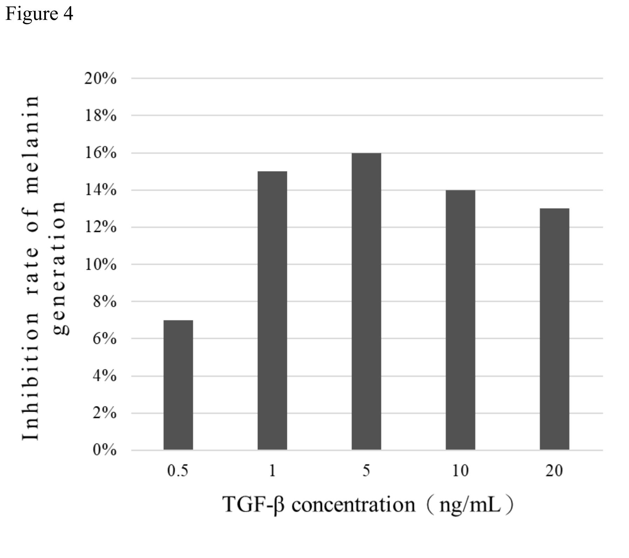

The experimental results are shown in table 4 and Figure 4. The amount of melanin in the control group is set as a benchmark. After calculations, when the TGF-β concentration reaches 0.5 ng/mL, the inhibition rate of melanin generation reaches 7%; when the TGF-β concentration reaches 1.0 ng/mL or more, the inhibition rate of melanin generation reaches 10% or more, and when the TGF-β concentration is in the range of 1.0 ng/mL to 10 ng/mL, the inhibition rate of melanin generation reaches 14% or even higher.

Table 4

| TGF-β concentration (ng/mL) | Melanin/cell | Inhibition rate of melanin generation (%) |
|---|---|---|
| 0 (control) | 1 | 0% |
| 0.5 | 0.93 | 7% |
| 1.0 | 0.85 | 15% |
| 5.0 | 0.84 | 16% |
| 10.0 | 0.86 | 14% |
| 20.0 | 0.87 | 13% |

### 5. Preparation of platelet dry powder

The platelet of the present invention is isolated from the whole blood of human or non-human mammals.

250 milliliters (mL) of whole blood was put into a blood bag containing an anticoagulant and centrifuged at 300 ~ 1,500×g for 3 to 10 minutes to obtain platelet-rich plasma (PRP), also known as plasma layer solution.

Centrifugation was performed again until platelets have collected at the bottom, removed the supernatant, so that the proteins of plasma were completely removed. Same volume of water for injection as the removed supernatant was added, and the purified platelets were obtained. These were then aliquoted into sample vials at 1 mL per vial. The vials were then freeze-dried to obtain the platelet powder.

### 6-1. Preparation of platelet culture medium

1 mL of melanocyte-specific basal cell culture medium (including melanocyte-specific growth factor kit) was used to dissolve platelet dry powder to prepare platelet dry powder medium. Each gram of growth factor dry powder contains $4\times10^8$ ~ $1.5\times10^{11}$platelets. The melanocyte-specific basal cell culture medium and platelet dry powder medium were mixed in a certain proportion to prepare melanocyte-specific culture medium containing different concentrations of platelet, namely: 0 platelet (control group), $1.25\times10^7$ platelets, $2.5\times10^7$ platelets, $5\times10^7$ platelets, $10\times10^7$ platelets, and $15\times10^7$ platelets.

### 6-2. The inhibition effect of platelet on generation of melanin

The effect of the amount of platelet on melanin was further investigated in the present invention.

The 96-well plate was divided into untreated wells (control group wells, 0 platelet), $1.25\times10^7$ platelets, $2.5\times10^7$ platelets, $5\times10^7$ platelets, $10\times10^7$ platelets, and $15\times10^7$ platelets, with 3 replicates per well.

HEM cells were added to untreated wells (control wells), treated wells with $1.25\times10^7$ platelets, $2.5\times10^7$ platelets, $5\times10^7$ platelets, $10\times10^7$ platelets, and $15\times10^7$ platelets, each well contained the same amounts of HEM cells, cells were cultured in an incubator at 37°C and 5% carbon dioxide ($CO_2$) for 24 hours. Remove the old culture medium (used medium), platelet dry powder culture medium corresponding to each group was added into the corresponding well and continued to incubate for 48 hours.

500 μL of TrypLE reagent (Gibco, A1285901) was added into each well cells, placed in an incubator at 37°C and 5% $CO_2$ for 3-5 minutes. After the reaction was completed, 500 μL of melanocyte-specific basal cell culture medium was added into each well to terminate the reaction of TrypLE reagent.

Cell fluid in each well was transferred into a microcentrifuge tube and centrifuged at 1,000-3,000 rpm (preferably, 2,000 rpm) for 10-30 minutes (preferably, 20 mins) with a microcentrifuge (Kubota, 3520) to remove the supernatant, and cell pellet was left. 110 μL of NaOH (Sigma, S5881) at a concentration of 1N was added into each tube for 10-20 minutes. After the reaction was completed, each mixture was transferred into a microcentrifuge tube and then analyzed using a multi-function microplate spectrophotometer (Varioskan LUX™, Thermo Scientific™) with absorbance value of 475 nm to obtain the absorbance of melanin.

The inhibition rate of melanin generation per unit of melanocytes was calculated as follows:

Inhibition rate of melanin generation (%) = (1 - the amount of melanin per unit of cell (tested group)) ÷ the amount of melanin per unit of cell (control group) × 100%

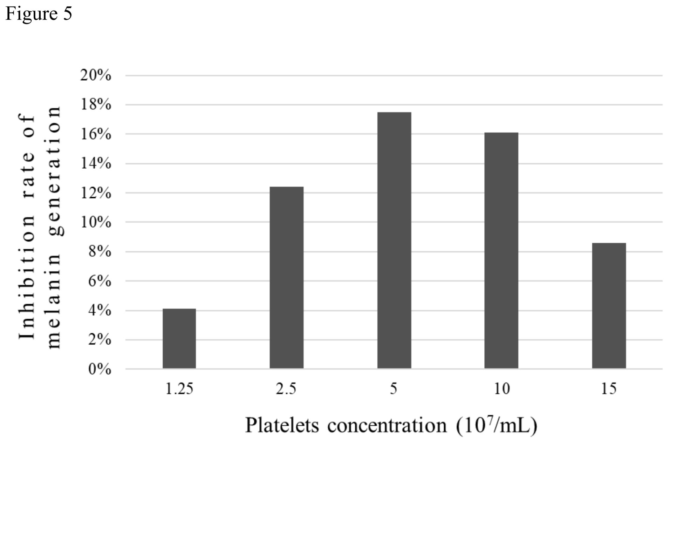

The experimental results are shown in table 5 and Figure 5. The amount of melanin in the control group is set as a benchmark. After calculations, when the platelet concentration is in the range of $2.5\times10^7$ to $15\times10^7$, the inhibition rate of melanin generation reaches 8% or more; When the platelet concentration is in the range of $2.5\times10^7$ to $10\times10^7$, the inhibition rate of melanin generation is as high as 12% or more, and when the platelet concentration is in the range of $5\times10^7$ to $10\times10^7$, the inhibition rate of melanin generation is as high as 16% or more.

Table 5

| Group | The number of melanocytes | The amounts of melanin | Melanin/Cell | Inhibition rate of melanin generation (%) |
|---|---|---|---|---|
| Control | 177,221 | 0.05515 | $3.11\times10^7$ | 0% |
| $1.25\times10^7$ | 209,321 | 0.0625 | $2.99\times10^7$ | 4.10% |
| $2.5\times10^7$ | 230,078 | 0.0627 | $2.73\times10^7$ | 12.40% |
| $5\times10^7$ | 268,700 | 0.069 | $2.57\times10^7$ | 17.50% |
| $10\times10^7$ | 294,707 | 0.07695 | $2.61\times10^7$ | 16.10% |
| $15\times10^7$ | 318,921 | 0.09075 | $2.85\times10^7$ | 8.60% |

**7-1. Preparation of platelet culture medium**

1 mL of melanocyte-specific basal cell culture medium (including melanocyte-specific growth factor kit) was used to dissolve platelet dry powder to prepare platelet dry powder medium. The melanocyte-specific basal cell culture medium and platelet dry powder medium were mixed in a certain proportion to prepare melanocyte-specific culture medium containing different concentrations of platelet, namely: 0 platelet (control group), $1.25\times10^7$ platelets, $2.5\times10^7$ platelets, $5\times10^7$ platelets, and $10\times10^7$ platelets.

**7-2. Preparation of melanocyte-specific culture medium containing different whitening ingredients**

Arbutin (Sigma, SI-A4256) was prepared with melanocyte-specific basal cell culture medium to achieve a final concentration of 50 μg/mL of arbutin. Glutathione (Sigma, SI-G6013) was prepared with melanocyte-specific basal cell culture medium to achieve a final concentration of 100 μg/mL of glutathione. Tranexamic acid (Siuguan, Taiwan FDA Pharmaceutical Manufacturing License No. 027356) was prepared with melanocyte-specific basal cell culture medium, so that the final concentration of Tranexamic acid was 2 mg/mL.

**7-3. The effect of platelets and different whitening ingredients on inflammation of melanocytes**

The 96-well plate was divided into control group wells (0 platelet), treated wells containing $1.25\times10^7$ platelets, $2.5\times10^7$ platelets, $5\times10^7$ platelets, and $10\times10^7$ platelets, Arbutin wells, Glutathione wells, and Tranexamic acid wells, with 3 replicates per well.

HEM cells were added into each well, separately, and each well contained the same amounts of HEM cells, cells were cultured in an incubator at 37°C and 5% carbon dioxide ($CO_2$) for 24 hours. Remove the old culture medium (used medium), melanocyte-specific culture medium prepared in steps 6-1 and 6-2 corresponding to each group was added into the corresponding well and continued to incubate for 48 hours.

The cell culture suspension in each well was transferred into a microcentrifuge tube. The concentration of Tumor Necrosis Factor-α (TNF-α) contained in the cell culture suspension was detected by using human TNF-α ELISA kit (abcam, ab181421) and analyzed by using a multi-function microplate spectrophotometer (Varioskan LUX™, Thermo Scientific™) with absorbance value of 450 nm. The regression curve of absorbance value and TNF-α was established according to the TNF-α ELISA kit product manual, and the absorbance value was then converted into TNF-α concentration to evaluate the inhibition effect of inflammation of melanocytes. The inhibition rate was calculated as follows:

Inhibition rate of inflammation (%) = 1 - (TNF-α concentration (tested group) ÷ TNF-α concentration (control group)) × 100%

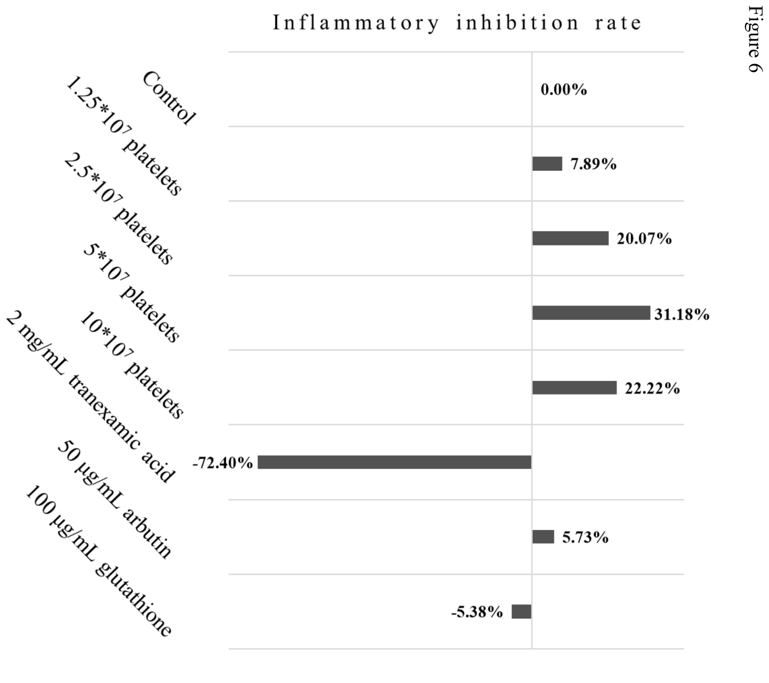

The experimental results are shown in table 6 and Figure 6. The TNF-α concentration of control group is set as a benchmark. After calculations, it was found that each platelet group has the ability to alleviate inflammation and is significantly better than common whitening ingredients on the market.

Table 6

| Group | TNF-α (pg/mL) | Inflammatory inhibition rate (%) |
|---|---|---|
| Control | 27.9 | 0% |
| $1.25\times10^7$ platelets | 25.7 | 7.89% |
| $2.5\times10^7$ platelets | 22.3 | 20.07% |
| $5\times10^7$ platelets | 19.2 | 31.18% |
| $10\times10^7$ platelets | 21.7 | 22.22% |
| 2 mg/mL tranexamic acid | 48.1 | -72.40% |
| 50 μg/mL arbutin | 26.3 | 5.73% |
| 100 μg/mL glutathione | 29.4 | -5.38% |

### 8-1. Preparation of platelet culture medium

1 mL of melanocyte-specific basal cell culture medium (including melanocyte-specific growth factor kit) was used to dissolve platelet dry powder to prepare platelet dry powder medium. The melanocyte-specific basal cell culture medium and platelet dry powder medium were mixed in a certain proportion to prepare melanocyte-specific culture medium containing different concentrations of platelet, namely: 0 platelet (control group), $1.25\times10^7$ platelets, $2.5\times10^7$ platelets, $5\times10^7$ platelets, and $10\times10^7$ platelets.

### 8-2. Preparation of melanocyte-specific culture medium containing different whitening ingredients

Arbutin (Sigma, SI-A4256) was prepared with melanocyte-specific basal cell culture medium to achieve a final concentration of 50 μg/mL of arbutin. Glutathione (Sigma, SI-G6013) was prepared with melanocyte-specific basal cell culture medium to achieve a final concentration of 100 μg/mL of glutathione. Tranexamic acid (Siuguan, Taiwan FDA Pharmaceutical Manufacturing License No. 027356) was prepared with melanocyte-specific basal cell culture medium, so that the final concentration of Tranexamic acid was 2 mg/mL.

### 8-3. The effect of platelets and different whitening ingredients on tyrosinase activity

The 96-well plate was divided into control group wells (0 platelet), treated wells containing $1.25\times10^7$ platelets, $2.5\times10^7$ platelets, $5\times10^7$ platelets, and $10\times10^7$ platelets, Arbutin wells, Glutathione wells, and Tranexamic acid wells, with 3 replicates per well.

HEM cells were added into each well, separately, and each well contained the same amounts of HEM cells, cells were cultured in an incubator at 37°C and 5% carbon dioxide ($CO_2$) for 24 hours. Remove the old culture medium (used medium), melanocyte-specific culture medium prepared in steps 7-1 and 7-2 corresponding to each group was added into the corresponding well and continued to incubate for 48 hours.

The cell culture suspension in each well was transferred into a microcentrifuge tube. The concentration of tyrosinase contained in the cell culture suspension was detected by using human tyrosinase ELISA kit (antibodies, A312872) and analyzed by using a multi-function microplate spectrophotometer (Varioskan LUX™, Thermo Scientific™) with absorbance value of 450 nm. The regression curve of absorbance value and tyrosinase was established according to the tyrosinase ELISA kit product manual, and the absorbance value was then converted into tyrosinase concentration to evaluate the inhibition effect of tyrosinase activity. The inhibition rate was calculated as follows:

Inhibition rate of tyrosinase (%) = 1 - (tyrosinase concentration (tested group) ÷ tyrosinase concentration (control group)) × 100%

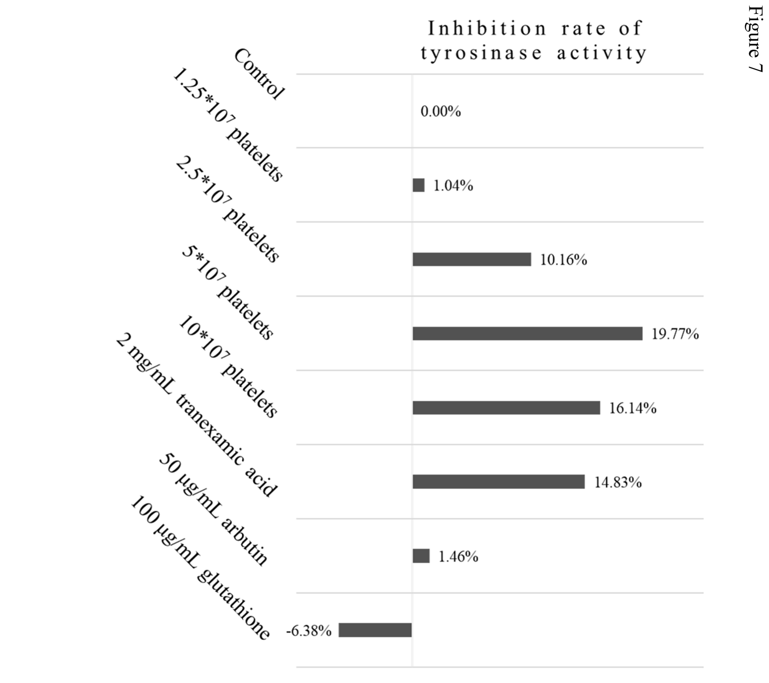

The experimental results are shown in table 7 and Figure 7. The tyrosinase concentration of control group is set as a benchmark for the calculation of inhibition rate of tyrosinase activity. After calculations, it was found that each platelet group has the ability to inhibit tyrosinase activity and is significantly better than common whitening ingredients on the market.

Table 7

| Group | Tyrosinase (pg/mL) | inhibition rate of tyrosinase activity (%) |
|---|---|---|
| Control | 327.8 | 0% |
| $1.25\times10^7$ platelets | 324.4 | 1.04% |
| $2.5\times10^7$ platelets | 294.5 | 10.16% |
| $5\times10^7$ platelets | 263.0 | 19.77% |
| $10\times10^7$ platelets | 274.9 | 16.14% |
| 2 mg/mL tranexamic acid | 279.2 | 14.83% |
| 50 μg/mL arbutin | 323.0 | 1.46% |
| 100 μg/mL glutathione | 348.7 | -6.38% |

## Claims

**1.** A composition for inhibiting generation of melanin, comprising platelets and/or growth factors isolated from blood.

**2.** The composition of claim 1, wherein the composition comprises platelets isolated from plasma.

**3.** The composition of claim 2, wherein the composition comprises $2.5\times10^7$ to $10\times10^9$ platelets.

**4.** The composition of claim 3, wherein the composition comprises $2.5\times10^7$ to $10\times10^8$ platelets.

**5.** The composition of claim **1,** wherein the growth factors is selected from platelet-derived growth factor-BB (PDGF-BB) or transforming growth factor (TGF-β).

**6.** The composition of claim 5, wherein the concentration of PDGF-BB is 0.1 ng/mL to 5 ng/mL.

**7.** The composition of claim 6, wherein the concentration of PDGF-BB is 0.1 ng/mL to 3 ng/mL.

**8.** The composition of claim 5, wherein the concentration of TGF-β is 0.5 ng/mL to 50 ng/mL.

**9.** The composition of claim 8, wherein the concentration of TGF-β is 0.5 ng/mL to 30 ng/mL.

**10.** The composition of claim 1, wherein the composition is a dry powder prepared by freeze drying.

**11.** The composition of claim 10, wherein the dry powder comprises $4\times10^8$ ~ $1.5\times10^{11}$ platelets per gram.

**12.** The composition of claim 10, wherein the dry powder comprises 15 to 850 ng of PDGF-BB per gram.

**13.** The composition of claim 10, wherein the dry powder comprises 75 to 8,500 ng of TGF-β per gram.

**14.** A composition according to any one of claims 1 to 13 for use in alleviating skin pigmentation in a subject.

**15.** The composition for use of claim 14, wherein the skin pigmentation comprises melasma.

Figure 1

Proliferation rate of melanocytes
180%
160%
140%
120%
100%
80%
60%
40%
20%
0%
100%
112%
123%
131%
139%
153%
0 ng/mL (Control)
0.12 ng/mL
0.25 ng/mL
0.5 ng/mL
1.0 ng/mL
2.0 ng/mL
PDGF-BB concentration

Figure 2

140%
120%
100%
80%
60%
40%
20%
0%
Generation rate of melanin
100%
104%
105%
110%
119%
133%
0 ng/mL (Control)
0.12 ng/mL
0.25 ng/mL
0.5 ng/mL
1.0 ng/mL
2.0 ng/mL
PDGF-BB concentration

Figure 3

20%
18%
16%
14%
12%
10%
8%
6%
4%
2%
0%
Inhibition rate of melanin generation
0.12
0.25
0.5
1
2
PDGF-BB concentration（ng/mL）

Figure 4

20%
18%
16%
14%
12%
10%
8%
6%
4%
2%
0%
Inhibition rate of melanin generation
0.5
1
5
10
20
TGF-β concentration（ng/mL）

Figure 5

20%
18%
16%
14%
12%
10%
8%
6%
4%
2%
0%
Inhibition rate of melanin generation
1.25
2.5
5
10
15
Platelets concentration (10^7/mL)

Figure 6

Inflammatory inhibition rate
0.00%
7.89%
20.07%
31.18%
22.22%
-72.40%
5.73%
-5.38%
Control
1.25*$10^7$ platelets
2.5*$10^7$ platelets
5*$10^7$ platelets
10*$10^7$ platelets
2 mg/mL tranexamic acid
50 μg/mL arbutin
100 μg/mL glutathione

Figure 7

Inhibition rate of tyrosinase activity
0.00%
1.04%
10.16%
19.77%
16.14%
14.83%
1.46%
-6.38%
Control
1.25*10⁷ platelets
2.5*10⁷ platelets
5*10⁷ platelets
10*10⁷ platelets
2 mg/mL tranexamic acid
50 μg/mL arbutin
100 μg/mL glutathione

# EUROPEAN SEARCH REPORT

Application Number
EP 25 22 2475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 103 524 613 A (SRGF BIOTECH CO LTD) 22 January 2014 (2014-01-22)<br>* claim 1 *<br>* page 1, paragraph 0002 *<br>* page 6, paragraph 0070 *<br>----- | 1-15 | INV.<br>A61K38/18<br>A61K35/19<br>A61Q19/02<br>A61P17/00 |
| X | US 2013/045270 A1 (MANTYLA EINAR [IS] ET AL) 21 February 2013 (2013-02-21)<br>* claims 1, 3, 21 *<br>* page 1, paragraph 0016 *<br>* page 2, paragraph 0026 *<br>* page 5, paragraph 0074 *<br>----- | 1,5,14,15 | |
| X | DATABASE EMBASE [Online] ELSEVIER SCIENCE PUBLISHERS, AMSTERDAM, NL;<br>1 March 2020 (2020-03-01),<br>TUKNAYAT A ET AL: "Clinical efficacy of platelet rich plasma in melasma",<br>XP002821917,<br>Database accession no. EMB-634422929<br>* abstract *<br>----- | 1,2,5,14,15 | |
| | | | TECHNICAL FIELDS SEARCHED (IPC) |
| X | TUKNAYAT ANKITA ET AL: "Platelet-rich plasma is a promising therapy for melasma",<br>JOURNAL OF COSMETIC DERMATOLOGY,<br>vol. 20, no. 8, 3 June 2021 (2021-06-03),<br>pages 2431-2436, XP093394499,<br>GB<br>ISSN: 1473-2130, DOI: 10.1111/jocd.14229<br>* abstract *<br>* page 2, left-hand column, paragraph 3 *<br>-----<br>-/-- | 1,2,5,14,15 | A61K<br>A61P<br>A61Q |

The present search report has been drawn up for all claims

| Place of search | Date of completion of the search | Examiner |
|---|---|---|
| Munich | 6 May 2026 | Virreira Winter, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

4

Europäisches
Patentamt
European
Patent Office
Office européen
des brevets

# EUROPEAN SEARCH REPORT

Application Number
EP 25 22 2475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PASINEE DEEUDOMWONGSA ET AL: "Exploring the potential of intradermal platelet-rich plasma in treating acquired bilateral nevus of Ota-like macule (Hori's nevus): A pilot study", JOURNAL OF COSMETIC DERMATOLOGY BLACKWELL SCIENCE, OXFORD, GB, vol. 23, no. 3, 13 December 2023 (2023-12-13), pages 803-811, XP072590693, ISSN: 1473-2130, DOI: 10.1111/JOCD.16135 * abstract * ----- | 1,2,14 | |
| A | MERCHÁN WILLIAM H. ET AL: "Platelet-rich plasma, a powerful tool in dermatology", JOURNAL OF TISSUE ENGINEERING AND REGENERATIVE MEDICINE, vol. 13, no. 5, 9 April 2019 (2019-04-09), pages 892-901, XP093394501, US ISSN: 1932-6254, DOI: 10.1002/term.2832 * abstract * * page 2; table 1 * ----- | 1-15 | |

TECHNICAL FIELDS SEARCHED (IPC)

The present search report has been drawn up for all claims

| Place of search | Date of completion of the search | Examiner |
|---|---|---|
| Munich | 6 May 2026 | Virreira Winter, N |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

4

# ANNEX TO THE EUROPEAN SEARCH REPORT ON EUROPEAN PATENT APPLICATION NO.

EP 25 22 2475

This annex lists the patent family members relating to the patent documents cited in the above-mentioned European search report. The members are as contained in the European Patent Office EDP file on
The European Patent Office is in no way liable for these particulars which are merely given for the purpose of information.

06-05-2026

| Patent document cited in search report | Publication date | Patent family member(s) | Publication date |
|---|---|---|---|
| CN 103524613 A | 22-01-2014 | NONE | |
| US 2013045270 A1 | 21-02-2013 | US 2013045270 A1 | 21-02-2013 |
| | | WO 2013005234 A1 | 10-01-2013 |

EPO FORM P0459

For more details about this annex : see Official Journal of the European Patent Office, No. 12/82